# EUROPEAN PATENT APPLICATION

(11) **EP 4 817 624 A2**
(43) Date of publication of application: **07.10.2026**
(21) Application number: 26200469.0
(22) Date of filing: 19.07.2022
(51) Int. Cl.: A61K 38/03

(54) **CYSTEINE REACTIVE PEPTIDES**

(30) Priority: 21.07.2021 US 202163224160 P
(62) Document number(s) of the earlier application(s) in accordance with Art. 76 EPC: 22846528.2 / 4 536 177
(71) Applicant: Purvala Bioscience, Inc., Cambridge, MA 02142 (US); Paloni, Justin, Natick, MA 01760 (US)
(72) Inventor: Paloni, Justin, Natick, MA 01760 (US)
(74) Representative: WP Thompson

(57) **Abstract**

Disclosed is a cysteine reactive functionalized amino add of the formula [X_{(*a*)} - L_{(*b*)} - Y_{(*c*)}]_{_{(*d*)}} where X and Y are the same or different, L is a peptide linker, a and c are an integer ≥ 0 and ≤10, wherein, a and c cannot both be 0; and b and d are an integer ≥ 1 and ≤ 20, cysteine reactive peptide are used for treating damaged keratin fibers.

## Description

### TECHNICAL FIELD

This application relates to cysteine reactive peptides and their use in compositions for treating and caring for keratins.

### BACKGROUND OF THE INVENTION

Keratin refers to the filament-forming proteins presenting specific physiochemical properties, which can be extracted from the cornified layer of the epidermis. Keratin is the main protein in skin and makes up hair, nails, and the surface layer of the skin. Harsh chemicals and environmental influences such as UV and thermal radiation lead to lasting keratin damage to skin, hair, and nails.

A keratin protein is defined by a primary structure based on amino acid chains. The chains vary in number and sequence of amino acids, polarity, charge, and size. Small modifications in the keratin's amino acid sequence cause significant property modification, since these sequences determine the whole molecular structure and the nature of the bonds. The sulphur-containing amino acids, methionine and cysteine establish intra or intermolecular disulfide bonds. The role of disulphide bonds is important in keratin's structural integrity. The disulfide bonds can be broken by chemical treatment of the hair and over time result in serious long-lasting damage to the keratin.

Thermoplastic polymer associations may lead to blend formation (physical blending) or copolymer formation (chemical blending) and offer temporary solutions to treat the damaged hair and skin. Chemical blending traditionally uses silicones and other conditioners that only provide surface treatments.

Different proteins developed to have useful functions and vary cell compatibility and mechanical properties. Natural in vivo associations between different proteins are found, for example in the blending between keratin and chitosan to form scaffolds and improve thermal stability. The biologically based composition containing cysteine reactive peptides disclosed herein would provide a more permanent solution to keratin damage and prevent adverse reactions found with the traditional chemical-based solutions.

### SUMMARY OF THE INVENTION

The object of the present invention is to provide a compound that may be used to treat or repair keratin fibers. The object is attained by providing a cysteine reactive peptide.

In a first aspect, the cysteine reactive peptide comprises a cysteine reactive functionalized amino acid of the formula [X_{(*a*)}-L_{(*b*)}-Y_{(*c*)}]_{(*d*)}, where X and Y are the same or different, L is a peptide linker, *a* and *c* are an integer ≥ 0 and < 10, wherein, a and *c* cannot both be 0; and *b* and *d* are an integer ≥ 1 and ≤ 20. The amino acid can be GGK. GKK, SEQ ID NO 3, SEQ ID NO 4, SEQ ID NO 5. SEQ ID NO 6, SEQ ID NO 7. SEQ ID NO 8 and SEQ ID NO 9 alone or in combination.

In a second aspect, the cysteine reactive peptide is used for treating damaged keratin fibers, comprising bringing the comprises a cysteine reactive functionalized amino acid of the formula [X_{(*a*)}-L_{(*b*)}-Y_{(*c*)}]_{(*d*)}, where X and Y are the same or different, L is a peptide linker, a and *c* are an integer ≥ 0 and < 10, wherein, *a* and *c* cannot both be 0; and *b* and *d* are an integer ≥ 1 and ≤ 20 damaged keratin fibers.

In a third aspect [X_{(*a*)}-L_{(*b*)}-Y_{(*c*)}] where X and Y are the same or different, L is a peptide linker, a and *c* are an integer ≥ 0 and < 10, wherein, *a* and *c* cannot both be 0; and *b* is an integer ≥ 1 and ≤ 20, and [X_{(*a*)}-L_{(*b*)}-Y_{(*c*)}] can occur in various combinations up to 20 times. The amino acid can be GGK, GKK, SEQ ID NO 3, SEQ ID NO 4, SEQ ID NO 5, SEQ ID NO 6, SEQ ID NO 7, SEQ ID NO 8 and (SEQ ID NO 9 alone or in combination. A nonlimiting example is [X_{(*a*)}-L_{(*b*)}- -Y_{(*c*)}][X'_{(*a*)}-L'_{(*b*)}-Y'_{(*c*)}][X"_{(*a*)}-L"_{(*b*)}-Y"_{(*c*)}]....up to 20 times.

### DESCRIPTION OF THE DRAWINGS

The following drawings form part of the present specification and are included to further demonstrate certain aspects of the claims.

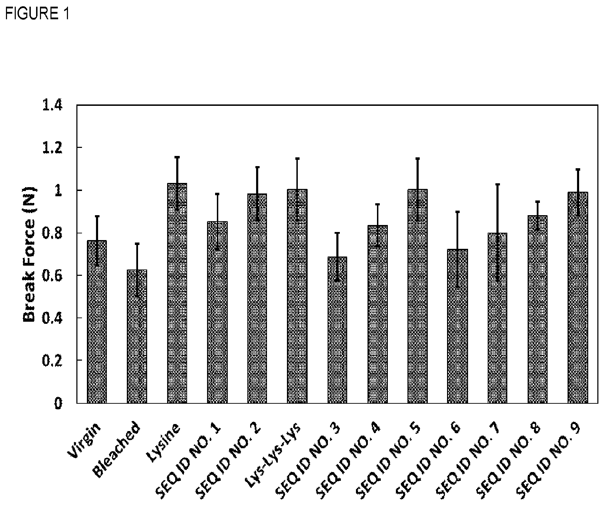

**FIG 1** **-** The y axis represents the break force in Newtons starting at 0, 0.2, 0.4, 0.6. 0.8, 1.0, 1.2, and 1.4. The x axis from left to right show hair treated as follows: virgin (untreated), bleached, lysine, SEQ ID NO 1, SEQ ID NO 2. Lys-Lys-Lys, SEQ ID NO 3, SEQ ID NO 4, SEQ ID NO 5, SEQ ID NO 6. SEQ ID NO 7, SEQ ID NO 8, AND SEQ ID NO 9. The error bars represent 95% confidence intervals.

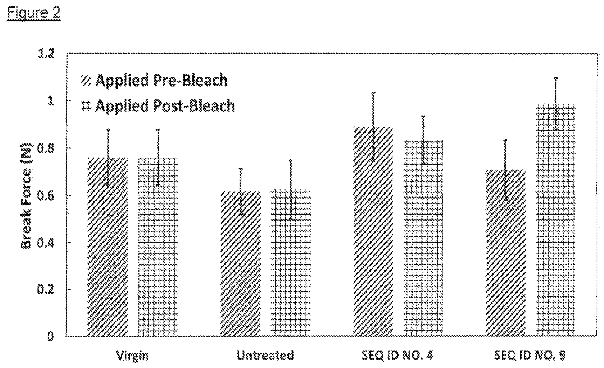

**FIG 2** - The y axis represents the break force in Newtons starting at 0, 0.2, 0.4, 0.6, 0.8. 1.0, and 1.2. The x axis from left to right compares pre-bleached hair (diagonal line pattern) versus post-bleach treated hair (square pattern). The hair moving from left to right are virgin (untreated with any additional treatment), SEQ ID NO 4 and SEQ ID NO 9. The error bars represent 95% confidence intervals.

### DETAILED DESCRIPTION

The present invention provides compounds useful for repairing damaged keratin fibers. The compounds can also provide protection to the keratin fibers to prevent future damage.

The terms used in this specification generally have their ordinary meanings in the art, within the context of the invention, and in the specific context where each term is used. Certain terms are discussed below. or elsewhere in the specification, to provide additional guidance to the practitioner in describing the compounds, compositions, and methods of the invention and how to make and use them. Moreover, it will be appreciated that the same thing can be said in more than one way. Consequently, alternative language and synonyms may be used for any one or more of the terms discussed herein, nor is any special significance to be placed upon whether or not a term is elaborated or discussed herein. The use of examples anywhere in this specification, including examples of any terms discussed herein, is illustrative only, and in no way limits the scope and meaning of the invention or of any exemplified term. Likewise, the invention is not limited to the examples presented.

As used herein, *"about"* or *"approximately"* shall generally mean within 20 percent, preferably within 10 percent, and more preferably within 5 percent of a given value or range. Other than in the operating examples, or where otherwise indicated, all numbers expressing quantities of ingredients and/or reaction conditions are to be understood as being modified in all instances by the term "about".

As used herein, the expression "at least one" means one or more and thus includes individual components as well as mixtures/combinations.

The reactive moieties described herein are linked via a linker. The term linker, as used herein, refers to one or more polyfunctional molecules which can be used to covalently couple the two or more reactive moieties without interfering with the reactive properties of the crosslinking agents. The peptide linkers referred to herein are any natural or non-natural amino acid that does not contain a reactive amine.

A thiol as discussed herein, is meant as an organosulfur compound R-S-H where R represents an alkyl or another organic substituent. The thiol groups are present on the cysteine residues within keratin.

Damaged keratin as used herein, means that the fibers comprising the keratin have broken disulphide bridges and free thiol groups are present on the keratin fiber.

Amino acids referred to herein are alpha amino acids.. Amino acids include naturally occurring amino acids and their derivatives. As used herein, amino acids with an extra carbon on the side chain are identified with the prefix "homo" and the conventional amino acid name.

The peptides referred to herein do not contain lipids. A peptide refers to two or more amino acids joined together by an amide bond
A hydrophobic group as used herein is a chemical group that is significantly non-polar and exhibits a tendency to dissolve in nonpolar solvents, such as hexane or toluene.

As used herein lipid refers to a straight chain hydrocarbon radical having 5 or more carbons and may comprise single, double, and/or triple bonds.

Lysines referred to herein are at least partially if not fully modified with cysteine- reactive functional groups.

When used in the context herein X means homocysteine.

As used herein SEQ ID NO 1 identifies a specific sequence of amino acids GGK.

As used herein SEQ ID NO 2 identifies a specific sequence of amino acids GKK

Hair as used herein refers at least one strand of hair from a human or animal. The hair may be natural and untreated (virgin hair) or processed, dyed, or bleached.

As used herein treating damaged keratin fibers requires the composition of formula 1 be applied to the keratin fiber either before or after damage has occurred. The keratin fiber may be virgin or damaged. The composition of formula 1 may be an ingredient in a carrier formulation. Examples of carrier formulations include, but are not limited to, creams, shampoos, oils, conditioners, masks, or any excipient carrier combination compatible with formula 1.

Functionalization of Amino Acids and Peptides with isothiocyanate modification of the process found in Sun, N.; Li, B.; Shao, J.; Mo, W.; Hu, B.; Shen, Z.; Hu, X. Beilstein J. Org. Chem. 2012, 8, 61-70, herein incorporated by reference. Functionalization of the amino acids herein are by isocyanate or isothiocyanate alone or in combination. A nonlimiting example of the amino acids used herein are arginine and lysine.

PREPARATION OF CYSTEINE REACTIVE PEPTIDES - 3 milligrams of lysine or a peptide corresponding to SEQ ID NO. 1, SEQ ID NO. 2. SEQ ID NO. 3, SEQ ID NO. 4, or SEQ ID NO. 5 was dissolved in 100 microliters of de-ionized water along with potassium carbonate (2 equivalents relative to the number of amines on lysine or the peptide). Then, carbon disulfide (1.2 equivalents relative to the number of amines on lysine or the peptide) was added dropwise, and the mixture was stirred at room temperature for 16 hours. After this time, 5 microliters of the reaction mixture were added to 1 microliter of 2% ninhydrin in ethanol. The solution was heated with a heat gun for 1 minute, and the solution color remained a faint yellow, indicating that no free amines were present. Next. the rection mixture was cooled to 0 °C, cyanuric chloride (0.5 equivalents relative to the number of amines on lysine or the peptide) in 50 microliters of acetonitrile was added, and the mixture was allowed to warm to room temperature while stirring. After 30 minutes, the reaction mixture was passed through a PD MiniTrap G-10 column using DI water as the elutant, and fractions containing the isothiocyanate-modified lysine or peptide were collected.

EXAMPLE - Swatches of Brazilian hair were bleached using BW2 hair powder lightener (Clariol, Stamford, CT) and oreor creme 40 volume developer (L'Oreal, Clichy, France) mixed in a 1:2 ratio with constant stirring for 1 minute until the mixture became smooth and homogeneous. The hair swatches were saturated in the bleaching mixture and left to sit at room temperature for 45 minutes. The hair swatches were then rinsed with deionized water (DI) for 2 minutes, thoroughly shampooed, and allowed to air dry. The bleaching procedure was repeated two additional times to yield bleached Brazilian hair swatches.

The bleached Brazilian hair swatches were cut into ½ inch wide samples and treated with 100 microliters of a 1 mg/mL solution in DI water of one of the isothiocyanate-modified lysine or peptide samples corresponding to SEQ ID NO. 1. SEQ ID NO. 2, SEQ ID NO. 3, SEQ ID NO. 4, or SEQ ID NO. 5 or unmodified peptides corresponding to SEQ ID NO. 6, SEQ ID NO. 7, SEQ ID NO. 8, or SEQ ID NO. 9. The treated hair samples were left to sit at room temperature for 30 minutes, after which time the hair samples were rinsed with DI water for 30 seconds, thoroughly shampooed, and allowed to air dry.

Tresses of Brazilian were cut into ½ inch wide samples shampooed and towel dried. The hair tresses were then treated with 100 microliters of a 1 mg/mL solution in DI water of the isothiocyanate-modified SEQ ID NO. 4 from Example 1 or the unmodified peptide SEQ ID NO. 9. The treated hair samples were left to sit at room temperature for 30 minutes, after which time the bleaching procedure from Example 2 was performed.

RESULTS - The break force of treated hair samples was measured using a custom apparatus. Individual hairs were cut into 13-centimeter-long sections. Both ends of the hairs were wrapped in tape 1 centimeter from each end. The hair was then held in a vertical position between two clamps (top = fixed, bottom = movable) with 1-centimeter-long grips such that the grips were aligned with the taped ends of the hair. Force was gradually applied to the bottom clamp, and the force at break was recorded. At least seven hair samples were collected for each treatment group.

For treated hair samples, all hair samples except for those treated with isothiocyanate-modified SEQ ID NO. 3 AND 4 or unmodified SEQ ID NO. 6 AND 7 showed a significant improvement in tensile strength over untreated bleached hair. Additionally, hair samples treated with isothiocyanate-modified lysine, as well as hair treated with unmodified SEQ ID NO. 9, displayed a significant improvement in tensile strength compared to untreated virgin hair.

For treated hair samples from Example 4. hair treated with isothiocyanate-modified SEQ ID NO. 4 did not show a significant difference in tensile strength from hair treated with the modified SEQ ID NO. 4 after bleaching. However, hair treated with unmodified SEQ ID. NO 9 had a significantly lower tensile strength compared to hair treated with the same peptide after bleaching.
This XML file does not appear to have any style information associated with it. The document tree is shown below. 
<ST26SequenceListing dtdVersion="V1_3" fileName="CYSTEINE REACTIVE PEPTIDES.xml" softwareName="WIPO
Sequence" softwareVersion="2.1.1" productionDate="2022-07-19">
  <ApplicationIdentification>
    <IPOfficeCode>US</IPOfficeCode>
    <ApplicationNumberText>63224160</ApplicationNumberText>
    <FilingDate>2021-07-21</FilingDate>
  </ApplicationIdentification>
  <ApplicantFileReference>0103-2010</ApplicantFileReference>
  <EarliestPriorityApplicationIdentification>
    <IPOfficeCode>US</IPOfficeCode>
    <ApplicationNumberText>63/224,160</ApplicationNumberText>
    <FilingDate>2021-07-21</FilingDate>
  </EarliestPriorityApplicationIdentification>
  <ApplicantName languageCode="en">PURVALA BIOSCIENCE, INC.</ApplicantName>
  <InventionTitle languageCode="en">CYSTEIN REATIVE PEPTIDES</InventionTitle>
  <SequenceTotalQuantity>9</SequenceTotalQuantity>
  <SequenceData sequenceIDNumber="1">
    <INSDSeq>
      <INSDSeq_length/>
      <INSDSeq_moltype/>
      <INSDSeq_division/>
      <INSDSeq_sequence>000</INSDSeq_sequence>
    </INSDSeq>
  </SequenceData>
  <SequenceData sequenceIDNumber="2">
    <INSDSeq>
      <INSDSeq_length/>
      <INSDSeq_moltype/>
      <INSDSeq_division/>
      <INSDSeq_sequence>000</INSDSeq_sequence>
    </INSDSeq>
  </SequenceData>
  <SequenceData sequenceIDNumber="3">
    <INSDSeq>
      <INSDSeq_length>5</INSDSeq_length>
      <INSDSeq_moltype>AA</INSDSeq_moltype>
      <INSDSeq_division>PAT</INSDSeq_division>
      <INSDSeq_feature-table>
        <INSDFeature>
          <INSDFeature_key>source</INSDFeature_key>
          <INSDFeature_location>1..5</INSDFeature_location>
          <INSDFeature_quals>
            <INSDQualifier>
              <INSDQualifier_name>mol_type</INSDQualifier_name>
              <INSDQualifier_value>protein</INSDQualifier_value>
            </INSDQualifier>
            <INSDQualifier id="q4">
              <INSDQualifier_name>organism</INSDQualifier_name>
              <INSDQualifier_value>synthetic construct</INSDQualifier_value>
            </INSDQualifier>
          </INSDFeature_quals>
        </INSDFeature>
      </INSDSeq_feature-table>
      <INSDSeq_sequence>GGGGK</INSDSeq_sequence>
    </INSDSeq>
  </SequenceData>
  <SequenceData sequenceIDNumber="4">
    <INSDSeq>
      <INSDSeq_length>9</INSDSeq_length>
      <INSDSeq_moltype>AA</INSDSeq_moltype>
 
<INSDSeq_division>PAT</INSDSeq_division>
<INSDSeq_feature-table>
<INSDFeature>
<INSDFeature_key>source</INSDFeature_key>
<INSDFeature_location>1..9</INSDFeature_location>
<INSDFeature_quals>
<INSDQualifier>
<INSDQualifier_name>mol_type</INSDQualifier_name>
<INSDQualifier_value>protein</INSDQualifier_value>
</INSDQualifier>
<INSDQualifier id="q5">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>GGGGGGGGK</INSDSeq_sequence>
</INSDSeq>
</SequenceData>
<SequenceData sequenceIDNumber="5">
<INSDSeq>
<INSDSeq_length>9</INSDSeq_length>
<INSDSeq_moltype>AA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..9</INSDFeature_location>
<INSDFeature_quals>
<INSDQualifier>
<INSDQualifier_name>mol_type</INSDQualifier_name>
<INSDQualifier_value>protein</INSDQualifier_value>
</INSDQualifier>
<INSDQualifier id="q6">
<INSDQualifier_name>organism</INSDQualifier_name>
<INSDQualifier_value>synthetic construct</INSDQualifier_value>
</INSDQualifier>
</INSDFeature_quals>
</INSDFeature>
</INSDSeq_feature-table>
<INSDSeq_sequence>IIIGGGGGK</INSDSeq_sequence>
</INSDSeq>
</SequenceData>
<SequenceData sequenceIDNumber="6">
<INSDSeq>
<INSDSeq_length/>
<INSDSeq_moltype/>
<INSDSeq_division/>
<INSDSeq_sequence>000</INSDSeq_sequence>
</INSDSeq>
</SequenceData>
<SequenceData sequenceIDNumber="7">
<INSDSeq>
<INSDSeq_length>6</INSDSeq_length>
<INSDSeq_moltype>AA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..6</INSDFeature_location>
<INSDFeature_quals>
<INSDQualifier>
<INSDQualifier_name>mol_type</INSDQualifier_name>
<INSDQualifier_value>protein</INSDQualifier_value>
 
</INSDQualifier>
          <INSDQualifier id="q8">
            <INSDQualifier_name>organism</INSDQualifier_name>
            <INSDQualifier_value>synthetic construct</INSDQualifier_value>
          </INSDQualifier>
        </INSDFeature_quals>
      </INSDFeature>
    </INSDSeq_feature-table>
    <INSDSeq_sequence>XGGGGX</INSDSeq_sequence>
  </INSDSeq>
</SequenceData>
<SequenceData sequenceIDNumber="8">
  <INSDSeq>
    <INSDSeq_length>8</INSDSeq_length>
    <INSDSeq_moltype>AA</INSDSeq_moltype>
    <INSDSeq_division>PAT</INSDSeq_division>
    <INSDSeq_feature-table>
      <INSDFeature>
        <INSDFeature_key>source</INSDFeature_key>
        <INSDFeature_location>1..8</INSDFeature_location>
        <INSDFeature_quals>
          <INSDQualifier>
            <INSDQualifier_name>mol_type</INSDQualifier_name>
            <INSDQualifier_value>protein</INSDQualifier_value>
          </INSDQualifier>
          <INSDQualifier id="q9">
            <INSDQualifier_name>organism</INSDQualifier_name>
            <INSDQualifier_value>synthetic construct</INSDQualifier_value>
          </INSDQualifier>
        </INSDFeature_quals>
      </INSDFeature>
    </INSDSeq_feature-table>
    <INSDSeq_sequence>XGGGGGGX</INSDSeq_sequence>
  </INSDSeq>
</SequenceData>
<SequenceData sequenceIDNumber="9">
  <INSDSeq>
    <INSDSeq_length>10</INSDSeq_length>
    <INSDSeq_moltype>AA</INSDSeq_moltype>
    <INSDSeq_division>PAT</INSDSeq_division>
    <INSDSeq_feature-table>
      <INSDFeature>
        <INSDFeature_key>source</INSDFeature_key>
        <INSDFeature_location>1..10</INSDFeature_location>
        <INSDFeature_quals>
          <INSDQualifier>
            <INSDQualifier_name>mol_type</INSDQualifier_name>
            <INSDQualifier_value>protein</INSDQualifier_value>
          </INSDQualifier>
          <INSDQualifier id="q10">
            <INSDQualifier_name>organism</INSDQualifier_name>
            <INSDQualifier_value>synthetic construct</INSDQualifier_value>
          </INSDQualifier>
        </INSDFeature_quals>
      </INSDFeature>
    </INSDSeq_feature-table>
    <INSDSeq_sequence>XGGGGGGGGX</INSDSeq_sequence>
  </INSDSeq>
</SequenceData>
</ST26SequenceListing>
 
Length: 9
Molecule Type: AA
Features Location/Qualifiers:
- source, 1..9
> mol_type, protein
> organism, synthetic construct
Residues:
IIIGGGGGK 9

Sequence Number (ID): 6
Residues:
000

Sequence Number (ID): 7
Length: 6
Molecule Type: AA
Features Location/Qualifiers:
- source, 1..6
> mol_type, protein
> organism, synthetic construct
Residues:
XGGGGX 6

Sequence Number (ID): 8
Length: 8
Molecule Type: AA
Features Location/Qualifiers:
- source, 1..8
> mol_type, protein
> organism, synthetic construct
Residues:
XGGGGGGX 8

Sequence Number (ID): 9
Length: 10
Molecule Type: AA
Features Location/Qualifiers:
- source, 1..10
> mol_type, protein
> organism, synthetic construct
Residues:
XGGGGGGGGX 10
END
 
Sequence Listing Information:
DTD Version: V1_3
File Name: CYSTEINE REACTIVE PEPTIDES.xml
Software Name: WIPO Sequence
Software Version: 2.1.1
Production Date: 2022-07-19
General Information:
Current application / IP Office: US
Current application / Application number: 63224160
Current application / Filing date: 2021-07-21
Current application / Applicant file reference: 0103-2010
Earliest priority application / IP Office: US
Earliest priority application / Application number: 63/224,160
Earliest priority application / Filing date: 2021-07-21
Applicant name: PURVALA BIOSCIENCE, INC.
Applicant name / Language: en
Invention title: CYSTEIN REATIVE PEPTIDES ( en )
Sequence Total Quantity: 9
Sequences:
Sequence Number (ID): 1
Residues:
000

Sequence Number (ID): 2
Residues:
000

Sequence Number (ID): 3
Length: 5
Molecule Type: AA
Features Location/Qualifiers:
- source, 1..5
> mol_type, protein
> organism, synthetic construct
Residues:
GGGGK 5

Sequence Number (ID): 4
Length: 9
Molecule Type: AA
Features Location/Qualifiers:
- source, 1..9
> mol_type, protein
> organism, synthetic construct
Residues:
GGGGGGGGK 9

Sequence Number (ID): 5
 THE FOLLOWING STATEMENTS DESCRIBE SOME EMBODIMENTS OF THE INVENTION
A. A composition comprising Formula I:

   [X_{(*a*)} - L_{(*b*)} - *Y*_{(*c*)}]_{(*d*)} (I)

   wherein X and Y are a cysteine reactive functionalized amino acid, and X and Y are the same or different;
   L is a peptide linker;
   *a* and *c* are an integer ≥ 0 and s 10, wherein, a and c cannot both be 0; and
   *b* and *d* are an integer ≥ 1 and ≤ 20.
B. The composition of Statement A, wherein the amino acid comprises a side chain thiol binding amine.
C. The composition of Statement B, wherein the side chain thiol binding amine is selected from the group consisting of an arginine and a lysine.
D. The composition of Statement A, wherein the amino acid is functionalized with an isocyanate.
E. The composition of Statement A, wherein the at least one amino acid is functionalized with an isothiocyanate.
F. The composition of Statement A, wherein the peptide linker is selected from the group comprising at least one of a natural or non-natural amino acid that does not contain a reactive amine.
G. The composition of Statement A, wherein the amino acid comprises GGK.
H. The composition of Statement A, wherein the amino acid comprises GKK.
I. The composition of Statement A, wherein the amino acid comprises SEQ ID NO 3.
J. The composition of Statement A, wherein the amino acid comprises SEQ ID NO 4.
K. The composition of Statement A, wherein the amino acid comprises SEQ ID NO 5.
L. The composition of Statement A, wherein the base amino add is homocysteine.
M. The composition of Statement A, wherein the amino acid comprises SEQ ID NO 8.
N. The composition of Statement A, wherein the amino acid comprises SEQ ID NO 7.
O. The composition of Statement A, wherein the amino acid comprises SEQ ID NO 8.
P. The composition of Statement A, wherein the amino acid comprises SEQ ID NO 9.
Q. The composition of Statement A, wherein the composition treats a keratin.
R. The composition of Statement Q, wherein the keratin is hair.
S. The composition of Statement Q, wherein the keratin is skin.
T. A method for treating damaged keratin fibers, comprising bringing the composition of Statement A in contact with damaged keratin fibers.
U. A composition comprising Formula I:

   [X_{(*a*)} - L_{(*b*)} - *Y*_{(*c*)}]_{(*d*)} (I)

   wherein X and Y are a cysteine reactive functionalized amino acid, and X and Y are the same or different;
   L is a peptide linker;
   *a* and *c* are an integer ≥ 0 and ≤ 10, wherein, a and c cannot both be 0;
   *b is* an integer ≥ 1 and ≤ 20; and
   [X_{(*a*)} - L_{(*b*)} - Y_{(*c*)}] can occur in various combinations up to 20 times.

## Claims

1. A method comprising applying a composition to keratin of a person, wherein the composition comprises an isothiocyanate-modified lysine or a derivative thereof.

2. A method comprising applying a composition to keratin of a person, wherein the composition comprises an isothiocyanate-modified lysine.

3. The method of claim 1 or claim 2, wherein the isothiocyanate-modified lysine is a diisothiocyanate-modified lysine.

4. The method of claim 3, wherein the diisothiocyanate-modified lysine has the following structure:

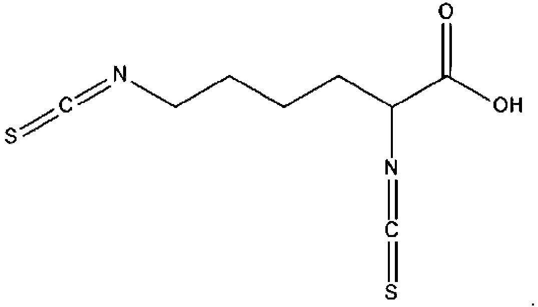

5. The method of claim 1 or claim 2, wherein the composition further comprises water.

6. The method of claim 1 or claim 2, wherein the keratin is in the person's hair.

7. The method of claim 6, wherein the person's hair is dyed or bleached prior to applying the composition to the person's hair.

8. The method of claim 6, wherein the person's hair is not dyed or bleached prior to applying the composition to the person's hair.

9. The method of claim 1 or claim 2, wherein the keratin is in the person's skin.
